# EUROPÄISCHE PATENTANMELDUNG

(11) **EP 4 624 036 A1**
(43) Veröffentlichungstag der Anmeldung: **01.10.2025**
(21) Anmeldenummer: 24167622.0
(22) Anmeldetag: 28.03.2024
(51) Int. Cl.: B01J 8/06, F28D 7/00

(54) **STÜTZPLATTE FÜR ROHRE IN EINEM REAKTORBEHÄLTER**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Oelmann, Tobias, 60439 Frankfurt (DE); Bartels, Katja, 60439 Frankfurt (DE); Müller, Brigitte, 60439 Frankfurt (DE); Gronemann, Veronika, 60439 Frankfurt (DE); Strozyk, Michael, 60439 Frankfurt (DE)
(74) Vertreter: Air Liquide

(57) **Zusammenfassung**

Vorrichtung (1.1; 1.2) umfassend einen Reaktorbehälter (2), ein Rohrbündel (3) mit mehreren Rohren (4), und zumindest eine Stützplatte (5), wobei das Rohrbündel (3) im Reaktorbehälter (2) angeordnet ist, wobei die Stützplatte (5) quer zu einer Längsachse (6) des Reaktorbehälters (2) im Reaktorbehälter (2) angeordnet ist, wobei jedes Rohr (4) des Rohrbündels (3) durch eine jeweilige Rohröffnung (7) der Stützplatte (5) geführt ist, wobei die Stützplatte (5) die Rohre (4) des Rohrbündels (3) in den Rohröffnungen (7) quer zur Längsrichtung der Rohre (4) stützt, wobei die Stützplatte (5) zwischen den Rohröffnungen (7) Ausschnitte (8) zum Fluidaustausch aufweist.

## Beschreibung

Die Erfindung betrifft eine Anordnung zur Methanolsynthese sowie eine Vorrichtung, welche Teil einer solchen Anordnung sein kann. Weiterhin betrifft die Erfindung eine Verwendung der Vorrichtung und ein Verfahren zur Methanolsynthese mit der Vorrichtung.

Bekannt sind Verfahren zur industriellen Herstellung von Methanol durch heterogen-katalytische Umsetzung von Synthesegas in geeigneten Synthesereaktoren. Synthesegase können unterschiedliche Gasgemische sein, die unter anderem Wasserstoff und Kohlenoxide enthalten. Der Reaktor ist meist als ein stehender Festrohrwärmetauscher ausgeführt.

Bekannt sind auch zweistufige Verfahren zur Herstellung von Methanol. Dabei wird Synthesegas einem wassergekühlten Reaktor und anschließend einem gasgekühlten Reaktor zugeführt. Mit einem kupferbasierten Festbett-Katalysator wird das Synthesegas zu Methanol umgesetzt.

Bei einem wassergekühlten Reaktor befindet sich der Katalysator innerhalb der Rohre, umgeben von Wasser oder Wasserdampf auf der Mantelseite. Die Rohre werden über Rohrplatten und Stützbleche im Reaktor mechanisch fixiert.

Die Kühlung im wassergekühlten Reaktor erfolgt über eine Wärmeabgabe ins Wasser, wobei Dampf entstehen kann. Das Dampf-Wasser-Gemisch steigt an den Rohren auf. Die Stützbleche müssen die Fixierung der Rohre sicherstellen. Bekannt sind Stützbleche, die versetzt im Reaktorbehälter eingesetzt sind, so dass das Kühlmittel mäanderförmig im Reaktorbehälter um die Stützbleche strömt.

Abhängig von den gewählten Geometrien der wärmeübertragenden Komponenten und der mechanischen Elemente der Kühlmittelseite wird ein Druckverlust auf der Kühlmittelseite im Wasserdampf erzeugt. Vor allem lange Reaktorrohre führen zu einem höheren Druckverlust.

Häufig sind die äußeren Abmessungen des Reaktors beim Transport beschränkt, so dass dies zu langen, schlanken Reaktoren führt. Daraus folgt jedoch ein erhöhter Druckverlust auf der Kühlmittelseite. Durch die lange Reaktorform werden weitere Stützplatten notwendig, um ein Durchhängen der Rohre bei Transport, sowie Durchbiegen, Knicken und Schwingen im Betrieb zu vermeiden.

Aufgabe der vorliegenden Erfindung ist es, ausgehend vom beschriebenen Stand der Technik die Hinderniswirkung der Stützplatte zu reduzieren und gleichzeitig ein Knicken, Durchbiegen oder Schwingen der Rohre zu verhindern.

Diese Aufgabe wird gelöst mit den unabhängigen Ansprüchen. Weitere vorteilhafte Ausgestaltungen sind in den abhängigen Ansprüchen angegeben. Die in den Ansprüchen und in der Beschreibung dargestellten Merkmale sind in beliebiger, technologisch sinnvoller Weise miteinander kombinierbar.

Erfindungsgemäß wird eine Vorrichtung vorgestellt, welche einen Reaktorbehälter, ein Rohrbündel mit mehreren Rohren, und zumindest eine Stützplatte umfasst. Das Rohrbündel ist im Reaktorbehälter angeordnet. Die Stützplatte ist quer zu einer Längsachse des Reaktorbehälters im Reaktorbehälter angeordnet und jedes Rohr des Rohrbündels ist durch eine jeweilige Rohröffnung der Stützplatte geführt. Dabei stützt die Stützplatte die Rohre des Rohrbündels in den Rohröffnungen quer zur Längsrichtung der Rohre. Weiterhin weist die Stützplatte zwischen den Rohröffnungen Ausschnitte zum Fluidaustausch auf.

Die Vorrichtung ist vorzugsweise als ein Reaktor ausgebildet. Die Vorrichtung kann zur Durchführung einer chemischen Reaktion eingerichtet sein. Bei der chemischen Reaktion kann es sich um eine exotherme oder endotherme Reaktion handeln. Besonders geeignet ist die Vorrichtung für die Methanolsynthese. Allerdings können die hierin beschriebenen Vorteile auch bei zahlreichen anderen chemischen Reaktionen erzielt werden. Die Vorteile können sogar erzielt werden, wenn die Vorrichtung nicht als Reaktor genutzt wird und in der Vorrichtung keine chemische Reaktion abläuft. Die Vorrichtung kann allgemein als Wärmetauscher ausgebildet sein.

Die Vorrichtung umfasst einen Reaktorbehälter und ein darin angeordnetes Rohrbündel mit mehreren Rohren. Durch die Rohre kann ein erstes Medium strömen. Außerhalb der Rohre kann sich ein zweites Medium in dem Reaktorbehälter befinden. Das zweite Medium kann durch Zwischenräume zwischen den Rohren strömen. Zwischen dem ersten Medium und dem zweiten Medium kann ein Wärmetausch stattfinden. Das erste Medium und das zweite Medium können in einander entgegengesetzte Richtungen strömen. In dem Fall wird die Vorrichtung im Gegenstrombetrieb betrieben. Das erste Medium und das zweite Medium können aber auch in die gleiche Richtung strömen. Im Falle der Methanolsynthese kann das erste Medium beispielsweise die Reaktionsedukte enthalten und das zweite Medium ein Kühlmedium sein, oder umgekehrt.

Vorzugsweise ist der Reaktorbehälter ein länglicher Hohlkörper. Der Reaktorbehälter kann ein zylindrischer Metallbehälter sein, der dazu geeignet ist, das Rohrbündel zu umfassen. Der Reaktorbehälter kann Anschlüsse und Verbindungen aufweisen, so dass das Rohrbündel fluidtechnisch angeschlossen werden kann. Weiterhin kann der Reaktorbehälter Anschlüsse und Verbindungen aufweisen, so dass in einen Mantelraum außerhalb und zwischen den Rohren ein Kühlmedium oder sonstiges Medium in den Reaktorbehälter eingeleitet und wieder abgeleitet werden kann. Der Mantelraum kann fluidtechnisch angeschlossen werden.

Das Rohrbündel umfasst mehrere Rohre. Das Rohrbündel umfasst vorzugsweise mindestens 6 Rohre. Bevorzugt umfasst das Rohrbündel mindestens 100 Rohre. Besonders bevorzugt umfasst das Rohrbündel sogar mindestens 1000 Rohre.

Die Rohre können längliche zylindrische Hohlkörper mit einer gleichmäßigen Wanddicke sein. Die Wanddicke der Rohre beträgt vorzugsweise höchstens 5 mm, bevorzugt höchstens 3 mm und besonders bevorzugt höchstens 0,1 mm. Die Rohre können insbesondere aus einem metallischen Material gefertigt sein. Bevorzugt ist das Material wärmeleitend.

Vorzugsweise beinhalten einige der Rohre oder alle Rohre einen Katalysator. Im Falle der Methanolsynthese können so beispielsweise die Reaktionsedukte durch die Rohre geleitet werden und in den Rohren mit dem Katalysator zur Reaktion gebracht werden, so dass Methanol entsteht.

Die Reaktionsedukte können insbesondere in Form eines Synthesegases bereitgestellt werden. Vorzugsweise sind Wasserstoff, Kohlenmonoxid und Kohlenstoffdioxid Reaktionsedukte. Bevorzugt ist ein Gemisch aus Wasserstoff und Kohlenmonoxid. Besonders bevorzugt ist ein Gemisch aus Wasserstoff und Kohlenstoffdioxid. Weiterhin kann das Synthesegas Inertgase enthalten. Insbesondere kann das Synthesegas Methan als Inertgas enthalten. Bevorzugt enthält das Synthesegas Stickstoff als Inertgas. Das Synthesegas kann auf einer Einlassseite in die Rohre geleitet werden und mit dem Katalysator im Rohr reagieren. Im Falle einer exothermen Reaktion kann bei der Reaktion entstehende Wärme über den Rohrmantel abgeleitet werden. Ein Kühlmittel kann die Wärme, bevorzugt konvektiv, von der Mantelseite abführen. Das im Rohr entstandene Produkt der Reaktion kann zusammen mit dem verbleibenden Synthesegas auf der Auslassseite abgeführt werden.

Alternativ kann ein Synthesegas mit den Reaktionsedukten auch außerhalb der Rohre durch den Reaktorbehälter geleitet werden. Insbesondere in dem Fall kann eine Katalysatorschüttung auf der Mantelseite im Mantelraum vorgesehen sein. Das Synthesegas kann dann durch die Katalysatorschüttung geführt werden. Ein Kühlmittel kann auf einer Einlassseite in die Rohre geleitet werden. Die durch die Reaktion erzeugte Wärme kann über die Mantelseite der Rohre an das Kühlmittel in den Rohren übertragen werden. Das erwärmte Kühlmittel kann auf einer Auslassseite der Rohre abgeführt werden.

Weiterhin umfasst die Vorrichtung die Stützplatte, über welche die Rohre in dem Reaktorbehälter gestützt sind. Die Stützplatte kann als ein Blech ausgebildet sein. Vorzugsweise hat die Stützplatte eine Dicke von mindestens 7 mm. Bevorzugt ist die Dicke der Stützplatte höchstens halb so groß wie ein Durchmesser der Rohre. Die optimale Dicke der Stützplatte kann darüber hinaus über Berechnungen ermittelt werden. Haben nicht alle Rohre den gleichen Durchmesser, ist die Dicke der Stützplatte vorzugsweise höchstens halb so groß wie der größte Durchmesser der Rohre des Rohrbündels.

Die Stützplatte ist quer zu einer Längsachse des Reaktorbehälters im Reaktorbehälter angeordnet. Die Längsachse des Reaktorbehälters ist bevorzugt entlang der länglichen Richtung des Hohlkörpers ausgerichtet. Vorzugsweise ist der Reaktorbehälter und/oder das Rohrbündel vertikal ausgerichtet. Die "und"-Ausführung ist bevorzugt.

Die Stützplatte weist Rohröffnungen zur Durchführung der Rohre des Rohrbündels auf. Jedes Rohr des Rohrbündels ist durch eine jeweilige Rohröffnung der Stützplatte geführt. Die Stützplatte stützt die Rohre des Rohrbündels in den Rohröffnungen quer zur Längsrichtung der Rohre.

Dies hat den Vorteil, dass die Rohre des Rohrbündels sich individuell thermisch ausdehnen können, aber Schwingungen durch bestimmte Strömungszustände und ein Knicken der Rohre vermieden werden können. Ein weiterer Vorteil ist, dass alle Rohre des Rohrbündels von einer Stützplatte gestützt sind. Eine unmittelbar benachbarte weitere Stützplatte, die verbleibende Rohre des Rohrbündels stützt, ist damit nicht notwendig.

Vorzugsweise sind die Rohre des Rohrbündels in axialer Richtung in der jeweiligen Rohröffnung der Stützplatte nicht fixiert. Die Rohre des Rohrbündels sind in dem Fall in axialer Richtung durch eine jeweilige Rohröffnung der Stützplatte axial bewegbar. Lokal unterschiedliche Temperaturen können lokal unterschiedliche Ausdehnungen der Rohre zur Folge haben. Dadurch, dass die Rohre axial bewegbar sind, werden einzelne Rohre nicht durch die Stützplatte in ihrer individuellen Ausdehnung gestört. Bewegungen quer zur Längsachse werden jedoch minimiert.

Die Form der Stützplatte kann der des Reaktorbehälters angepasst sein. Vorzugsweiseweist die Stützplatte eine rechteckige Form mit abgerundeten Ecken auf. Besonders bevorzugt weist die Stützplatte eine elliptische Form, insbesondere eine kreisförmige Form auf.

Die Stützplatte kann der Fluidaustausch zwischen dem oberhalb der Stützplatte gelegenen Teil des Reaktorbehälters und dem unterhalb der Stützplatte gelegenen Teil des Reaktorbehälters beeinträchtigen. Um diesen Effekt so gering wie möglich zu halten, weist die Stützplatte zwischen den Rohröffnungen Ausschnitte zum Fluidaustausch auf. Der Fluidaustausch findet insbesondere zwischen Bereichen des Reaktorbehälters statt, zwischen denen Stützplatte angeordnet ist. Der freie Strömungsquerschnitt im Reaktorbehälter kann durch die Ausschnitte in der Stützplatte bestimmt werden. Der freie Strömungsquerschnitt ist der Querschnitt eines Rohres oder Kanals, durch den ein Medium strömt. Der freie Strömungsquerschnitt bezeichnet also eine Fläche, durch die ein Medium strömen kann.

Je größer die gesamte Fläche der Ausschnitte ist, desto größer ist der freie Strömungsquerschnitt. Anderseits kann der freie Strömungsquerschnitt auch über die Teilung bestimmt werden. Die Teilung beschreibt den Abstand von Rohr zu Rohr. Je mehr Abstand zwischen Rohren ist, desto größer ist der freie Strömungsquerschnitt ohne eine Stützplatte. Die Teilung kann somit eine maximale Fläche der Ausschnitte definieren.

Bei einer festgelegten Anzahl an Ausschnitten, kann der freie Strömungsquerschnitt erhöht werden, indem die einzelnen Ausschnitte vergrößert werden.

Der Reaktorbehälter kann mehrere Stützplatten aufweisen. Vorzugsweise sind mehrere Stützplatten entlang der Längsachse im Reaktorbehälter verteilt. Die Stützplatten sind bevorzugt gleichmäßig entlang der Längsachse im Reaktorbehälter verteilt. Die Stützplatten sind vorzugsweise jeweils wie die hierin beschriebene Stützplatte ausgebildet. Es ist aber auch möglich, dass die Vorrichtung neben einer wie hierin beschrieben ausgebildeten Stützplatte eine oder mehrere weitere Stützplatten oder vergleichbare Elemente aufweist, welche nicht wie die hierin beschrieben ausgebildete Stützplatte ausgebildet sind.

Die Vorrichtung hat den Vorteil, dass die Hinderniswirkung der Stützplatte minimiert wird und gleichzeitig ein Knicken, Durchbiegen oder Schwingen der Rohre während des Transports oder während des Betriebs verhindert wird. Die Hinderniswirkung der Stützplatte kann minimiert werden, indem der freie Strömungsquerschnitt durch die Ausschnitte verbessert wird.

In einer bevorzugten Ausführungsform der Vorrichtung bildet die Stützplatte für zumindest einige der Ausschnitte zwischen dem Ausschnitt und den dazu nächstliegenden Rohröffnungen einen jeweiligen Steg aus. Die Stege haben jeweils eine minimale Breite, welche bei zumindest einigen der Stege, vorzugsweise bei allen der Stege gleich groß ist.

Die Stützplatte soll einerseits so stabil ausgebildet sein, dass die Rohre ausreichend gestützt werden. Dies kann dadurch erreicht werden, dass die Stege eine ausreichende Breite haben. Andererseits soll die Stützplatte den Fluidaustausch möglichst wenig beeinträchtigen. Dies kann dadurch erreicht werden, dass die Stege möglichst klein gehalten sind. Durch Abwägung der beiden genannten Bedingungen kann bestimmt werden, wie breit ein Steg an seiner engsten Stelle optimalerweise sein sollte, wie groß also die minimale Breite eines Steges optimalerweise sein sollte. In der vorliegenden Ausführungsform haben zumindest einige der der Stege, vorzugsweise sogar alle der Stege die gleiche minimale Breite. Damit kann für alle diese Stege ein bestmöglicher Kompromiss zwischen Stabilität und Fluidaustausch erzielt werden.

In einer weiteren bevorzugten Ausführungsform der Vorrichtung liegen zumindest einige der Ausschnitte jeweils zentral zwischen drei paarweise zueinander benachbarten der Rohröffnungen.

Die Ausschnitte können beispielsweise jeweils zentral zwischen drei benachbarten Rohröffnungen liegen, wobei die Mittelpunkte dieser drei Rohröffnungen bevorzugt ein gleichseitiges Dreieck bilden.

Vorzugsweise sind zumindest einige der Ausschnitte Durchgangsbohrungen. Der Vorteil von Durchgangsbohrungen ist, dass die Stützplatte besonders einfach hergestellt werden kann und eine sehr hohe Festigkeit und Steifigkeit aufweist.

In einer weiteren bevorzugten Ausführungsform der Vorrichtung weist die Stützplatte Zwischenbereiche auf, welche jeweils zwischen drei paarweise zueinander benachbarten der Rohröffnungen ausgebildet sind, und wobei zumindest einige der Ausschnitte der Stützplatte auf einen jeweiligen der Zwischenbereiche beschränkt sind.

Der Vorteil dieser Ausführungsform ist, dass bei gleichbleibender Teilung die Fläche des Ausschnitts erhöht werden kann. Bildlich gesprochen kann der Zwischenbereich zwischen den Rohröffnungen besser genutzt werden. Die Hinderniswirkung der Stützplatte kann somit weiter reduziert werden.

In einer weiteren bevorzugten Ausführungsform der Vorrichtung sind zumindest einige der Zwischenbereiche sternförmig.

Ein sternförmiger Zwischenbereich ist hier im mathematischen Sinne zu verstehen. Ein Zwischenbereich ist sternförmig, wenn es einen Punkt, den sog. Sternmittelpunkt, gibt, von dem aus alle weiteren Punkte des Zwischenbereichs sichtbar sind. Das heißt, dass jede Verbindungsstrecke eines Punktes mit dem Sternmittelpunkt vollständig in dem Zwischenbereich liegt. Der Sternmittelpunkt kann bildlich gesprochen alle Seiten des Zwischenbereichs "sehen."

Vorzugsweise können zumindest einige der Zwischenbereiche die Form von triangulären Löchern aufweisen. Ein trianguläres Loch meint hier ein dreieckiges Loch mit abgerundeten Ecken und konkaven Seiten. Ein trianguläres Loch ist sternförmig im hierin verwendeten Sinne des Begriffs sternförmig.

Der Vorteil dieser Ausführungsform ist, dass bei gleichbleibender Teilung die Fläche des Ausschnitts erhöht werden kann. Bildlich gesprochen kann der verfügbare Platz zwischen den Rohröffnungen besser genutzt werden. Die Hinderniswirkung der Stützplatte kann somit weiter reduziert werden.

In einer weiteren bevorzugten Ausführungsform der Vorrichtung weist die Stützplatte Zwischenbereiche auf, welche jeweils zwischen drei paarweise zueinander benachbarten der Rohröffnungen ausgebildet sind, und wobei sich zumindest einige der Ausschnitte der Stützplatte jeweils über mindestens zwei benachbarte der Zwischenbereiche erstrecken.

Diese Ausgestaltung kann erhalten werden, indem zwischen zwei benachbarten Zwischenbereichen jeweils die Verbindungsstege entfernt werden. Um eine Rohröffnung können in dieser Ausgestaltung beispielsweise drei Ausschnitte gebildet sein. Pro Ausschnitt können jeweils zwei benachbarte Zwischenbereiche verbunden werden. Jeder Ausschnitt kann vier benachbarte Rohröffnungen haben. Die vier Rohröffnungen können dabei als eine Gruppe eine Rautenform aufweisen.

Der Vorteil dieser Ausführungsform ist, dass die Hinderniswirkung der Stützplatte reduziert werden kann. Der freie Strömungsquerschnitt ist trotz einer geringen Teilung der Rohre verhältnismäßig hoch. Weiterhin kann die Stützplatte durch diese Ausführungsform Gewicht einsparen.

In einer weiteren bevorzugten Ausführungsform der Vorrichtung umgreift die Stützplatte alle Rohre des Rohrbündels.

Der Vorteil dieser Ausführungsform ist, dass die Stützplatte alle Rohre abstützt. Dadurch wird verhindert, dass einzelne Rohre schwingen oder durchbiegen während des Betriebs oder während des Transports.

Als ein weiterer Aspekt der Erfindung wird eine Anordnung zur Methanolsynthese vorgestellt. Die Anordnung umfasst die beschriebene Vorrichtung.

Die beschriebenen Vorteile und Merkmale der Vorrichtung sind auf die Anordnung anwendbar und übertragbar.

Zum Erzeugen von Methanol kann ein Synthesegas durch Umsetzen an Katalysatoren zu Methanol umgesetzt werden. Das Synthesegas kann Reaktionsedukte für die Methanolsynthese enthalten.

Neben der beschriebenen Vorrichtung umfasst die Anordnung vorzugsweise eine Quelle für Reaktionsedukte für die Methanolsynthese. Die Anordnung kann auch mehrere wie beschrieben ausgebildete Vorrichtungen aufweisen, welche beispielsweise in Reihe geschaltet sind.

Als ein weiterer Aspekt der Erfindung wird ein Verfahren vorgestellt. In dem Verfahren werden Reaktionsedukte für die Methanolsynthese durch die Rohre des Rohrbündels geleitet. Ein Kühlmedium wird außerhalb der Rohre des Rohrbündels durch den Reaktorbehälter geleitet. Vorzugsweise ist das Kühlmedium flüssiges Wasser und/oder Wasserdampf.

In einer alternativen Ausführung des Verfahrens können die Reaktionsedukte für die Methanolsynthese um die Rohre des Rohrbündels durch den Reaktorbehälter geleitet werden. Ein Kühlmedium wird dann durch die Rohre des Rohrbündels geleitet. Vorzugsweise ist das Kühlmedium gasförmig.

Die beschriebenen Vorteile und Merkmale der Vorrichtung sind auf das Verfahren anwendbar und übertragbar, und umgekehrt. Die Vorrichtung ist vorzugsweise zum Betrieb gemäß dem beschriebenen Verfahren eingerichtet. Das Verfahren wird vorzugsweise mit der Vorrichtung durchgeführt.

Als ein weiterer Aspekt der Erfindung wird eine Verwendung vorgestellt. Die beschriebene Vorrichtung wird dabei zur Methanolsynthese verwendet. Reaktionsedukte werden in der Vorrichtung zu Methanol umgesetzt.

Die beschriebenen Vorteile und Merkmale der Vorrichtung, der Anordnung und des Verfahrens sind auf die Verwendung anwendbar und übertragbar, und umgekehrt.

Vorzugsweise wird die Vorrichtung für die wassergekühlte Methanolsynthese verwendet. Alternativ ist bevorzugt, dass die Vorrichtung für die gasgekühlte Methanolsynthese verwendet wird.

In der wassergekühlten Methanolsynthese werden die Rohre mit Wasser gekühlt, während ein Synthesegas in den Rohren zu Methanol umgesetzt wird. Das Wasser führt die Wärme von den Rohren ab und verdampft dabei teilweise. Die aufsteigenden Dampfblasen können durch die Ausschnitte der Stützplatte strömen.

In der gasgekühlten Methanolsynthese werden die Rohre mit einem kühleren Gas in den Rohren gekühlt. Das Synthesegas wird außerhalb der Rohre zu Methanol umgesetzt. Dabei wird die Wärme an die Rohre übertragen und durch das Gas in den Rohren abgeführt. Das Synthesegas kann durch die Ausschnitte der Stützplatte strömen.

Die Erfindung wird nachfolgend anhand der Figuren näher erläutert. Die Figuren zeigen besonders bevorzugte Ausführungsbeispiele, auf welche die Erfindung jedoch nicht begrenzt ist. Die Figuren und die darin dargestellten Größenverhältnisse sind nur schematisch. Es zeigen:
- Fig. 1:: eine schematische Ansicht einer erfindungsgemäßen Anordnung zur Methanolsynthese,
- Fig. 2:: eine schematische Draufsicht auf eine erste Ausgestaltung einer Stützplatte, wie sie bei der Anordnung aus Fig. 1 verwendet werden kann,

- Fig. 3:: eine Schnittdarstellung der Stützplatte aus Fig. 2,
- Fig. 4:: eine Detailansicht einer zweiten Ausgestaltung einer Stützplatte, wie sie bei der Anordnung aus Fig. 1 verwendet werden kann,
- Fig. 5:: eine Detailansicht einer dritten Ausgestaltung einer Stützplatte, wie sie bei der Anordnung aus Fig. 1 verwendet werden kann,
- Fig. 6:: eine Detailansicht einer vierten Ausgestaltung einer Stützplatte, wie sie bei der Anordnung aus Fig. 1 verwendet werden kann.

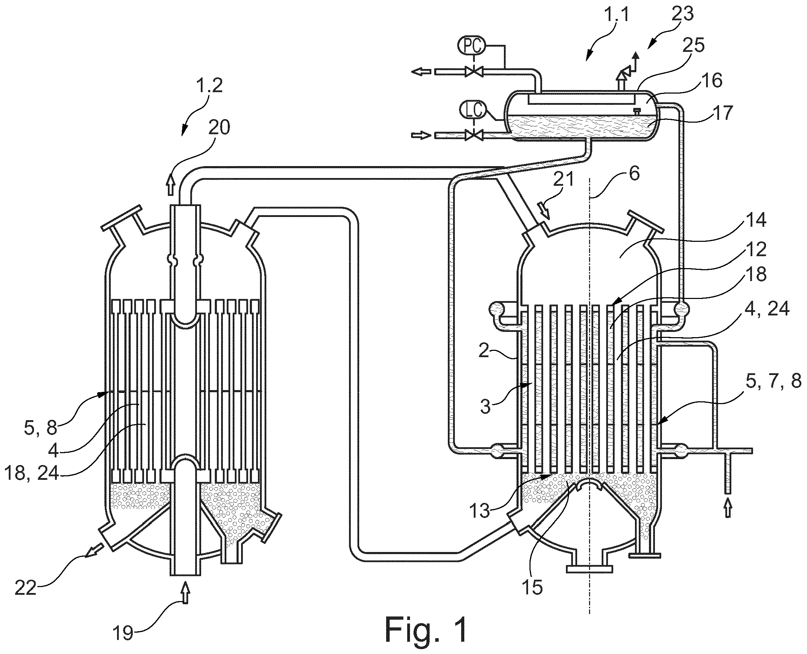

Fig. 1 zeigt eine schematische Ansicht einer Anordnung 23 zur Methanolsynthese. Dabei wird Synthesegas durch zwei in Serie geschaltete Vorrichtungen 1. 1; 1.2 geleitet. Die Vorrichtungen 1.1; 1.2 werden zur Methanolsynthese verwendet.

Vorgeheiztes Synthesegas wird durch einen Gaseinlass 21 der ersten Vorrichtung 1.1 zugeführt. Die erste Vorrichtung 1.1 umfasst einen Reaktorbehälter 2, ein Rohrbündel 3 mit mehreren Rohren 4 und mehrere Stützplatten 5. Das Rohrbündel 3 ist im Reaktorbehälter 2 angeordnet. Die Stützplatten 5 sind jeweils quer zu einer Längsachse 6 des Reaktorbehälters 2 im Reaktorbehälter 2 angeordnet, wobei jedes Rohr 4 des Rohrbündels 3 durch eine jeweilige Rohröffnung 7 der Stützplatten 5 geführt ist. Die Stützplatten 5 stützen die Rohre 4 des Rohrbündels 3 in den Rohröffnungen 7 quer zur Längsrichtung der Rohre 4. Zwischen den Rohröffnungen 7 weisen die Stützplatten Ausschnitte 8 zum Fluidaustausch auf.

Die Rohre 4 sind an einem ersten Ende 12 und an einem zweiten Ende 13 mit einer jeweiligen Rohrendplatte verbunden. Der Reaktorbehälter 2 und die Rohre 4 des Rohrbündels 3 sind aufrecht ausgerichtet. Das erste Ende 12 ist oben und das zweite Ende 13 ist unten im Reaktorbehälter 2 angeordnet.

Das Synthesegas wird in einem Verteiler 14 auf die Rohre 4 des Rohrbündels 3 verteilt.

In den Rohren 4 befindet sich ein Katalysator 24. Das Synthesegas enthält Reaktionsedukte. Die Reaktionsedukte für die Methanolsynthese werden durch die Rohre 4 des Rohrbündels 3 geleitet. Dabei wird das Synthesegas in den Rohren 4 teilweise zu Methanol umgesetzt. Ein Kühlmedium wird außerhalb der Rohre 4 des Rohrbündels 3 durch den Reaktorbehälter 2 geleitet. Die aus der exothermen Reaktion im Rohr 4 freiwerdende Wärme wird über die Rohrwand auf das in einem Mantelraum 18 befindliche Kühlmedium übertragen. Während der Katalysator 24 im Rohr 4 durch diesen Prozess gekühlt wird, wird dem Kühlmedium Energie zugeführt. Als Kühlmedium dient Wasser, welches dem Mantelraum 18 auf der zweiten Seite 13 zugeführt wird. Entstehende Dampfblasen und das siedende zweiphasige Wassergemisch strömen aufgrund von Dichteunterschieden vertikal nach oben. Dabei strömt der Wasserdampf durch die Ausschnitte 8 der Stützplatte 5 mit geringen Druckverlusten. Der Wasserdampf wird oben gesammelt und einem Wasser-Kondensator 25 zugeführt. In dem Reservoir des Kondensators 25 befinden sich gesättigter Dampf 16 und Wasser 17 am bzw. leicht unter dem Siedepunkt. Das Wasser 17 wird der ersten Vorrichtung 1.1 auf der unteren Seite 13 zugeführt und auf den Mantelraum 18 des Reaktorbehälters 2 verteilt. Die Kühlung funktioniert damit nach dem Thermosiphoneffekt.

Produktgas und das teilweise nicht reagierte Synthesegas werden aus dem Rohrbündel 3 in einem Sammler 15 gesammelt. Das Gasgemisch strömt anschließend zu der zweiten Vorrichtung 1.2.

In der zweiten Vorrichtung 1.2, welche der ersten Vorrichtung 1.1 nachgeschaltet ist, wird Synthesegas aus einem Synthesegaseinlass 19 vorgeheizt.

Die zweite Vorrichtung 1.2 ist teilweise analog zur ersten Vorrichtung 1.1 aufgebaut.

Im Unterschied zur ersten Vorrichtung 1.1, befindet sich in der zweiten Vorrichtung 1.2 zwischen den Rohren 4 der zweiten Vorrichtung 1.2 im Mantelraum 18 ein als Katalysatorschüttung vorliegender Katalysator 24.

Das Synthesegas, welches die Reaktionsedukte umfasst, wird für die Methanolsynthese außerhalb der Rohre 4 des Rohrbündels 3 durch den Reaktorbehälter 2 durch den Mantelraum 18 geleitet und ein Kühlmedium wird durch die Rohre 4 des Rohrbündels 3 geleitet. Dabei strömt das Synthesegas im Mantelraum 18 durch die Ausschnitte 8 der Stützplatten 5 mit geringen Druckverlusten nach unten zu einem Produktgasauslass 22.

Die aus der exothermen Reaktion im Mantelraum 18 freiwerdende Wärme wird über die Rohrwand auf das im Rohr 4 befindliche Kühlmedium übertragen. Während der Katalysator 24 im Mantelraum 18 durch diesen Prozess gekühlt wird, wird dem Kühlmedium Energie zugeführt. Als Kühlmedium dient Synthesegas, welches den Rohren 4 über den Synthesegaseinlass 19 zugeführt wird. Durch die zugeführte Energie erwärmt sich das Synthesegas aus dem Synthesegaseinlass 19 und wird als vorgeheiztes Synthesegas 20 zum Gaseinlass 21 der ersten Vorrichtung 1.1 geführt.

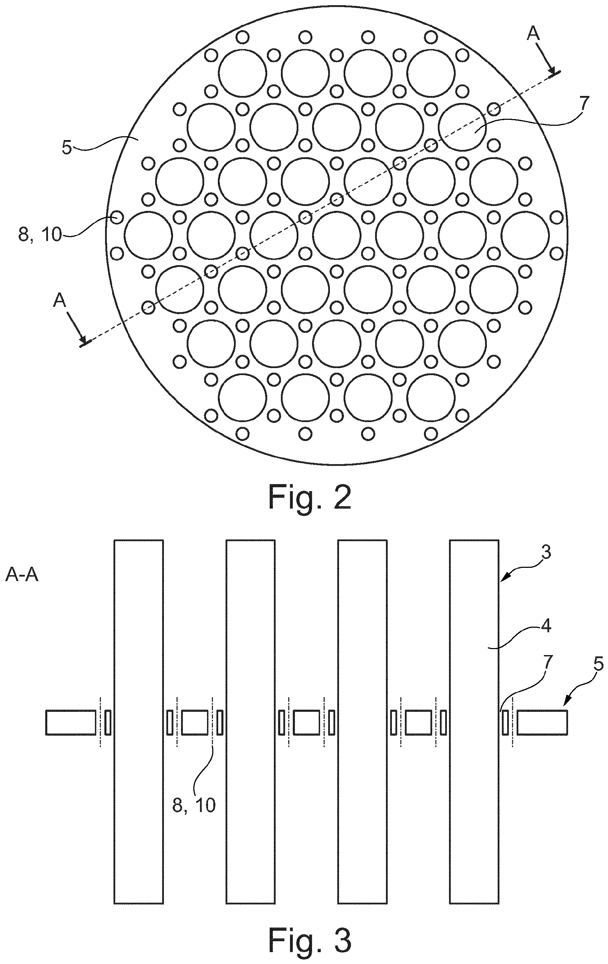

Fig. 2 zeigt eine schematische Draufsicht auf eine erste Ausgestaltung einer Stützplatte 5, wie sie in den Vorrichtungen 1.1;1.2 der Fig. 1 eingesetzt werden kann. Die Stützplatte 5 weist zwischen den Rohröffnungen 7 Ausschnitte 8 zum Fluidaustausch auf. Die Ausschnitte 8 sind als Durchgangsbohrungen 10 gefertigt.

Fig. 3 zeigt eine Schnittdarstellung der Stützplatte 5 aus Fig. 2. Jedes Rohr 4 des Rohrbündels 3 ist durch eine jeweilige Rohröffnung 7 der Stützplatte 5 geführt. Die Stützplatte 5 stützt die Rohre 4 des Rohrbündels 3 in den Rohröffnungen 7 quer zur Längsrichtung der Rohre 4.

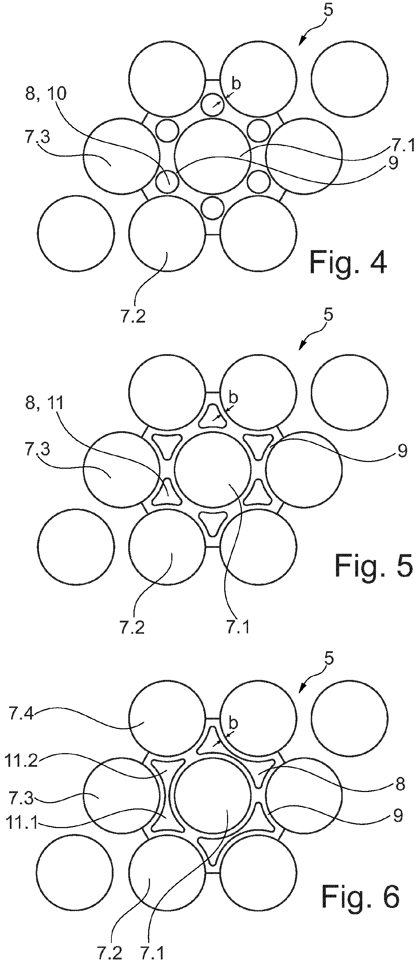

Fig. 4 zeigt eine Detailansicht einer zweiten Ausgestaltung einer Stützplatte 5, wie sie in den Vorrichtungen 1.1;1.2 der Fig. 1 eingesetzt werden kann. Die Stützplatte 5 bildet für zumindest einige der Ausschnitte 8 zwischen dem Ausschnitt 8 und den dazu nächstliegenden der Rohröffnungen 7.1 - 7.3 einen jeweiligen Steg 9 aus. Die Stege 9 haben jeweils eine minimale Breite b, welche bei zumindest einigen der Stege 9 gleich groß ist. Die Ausschnitte 8 liegen jeweils zentral zwischen drei paarweise zueinander benachbarten der Rohröffnungen 7.1 - 7.3. Die Ausschnitte 8 sind als Durchgangsbohrungen 10 gefertigt.

Fig. 5 zeigt eine Detailansicht einer dritten Ausgestaltung einer Stützplatte 5, wie sie in den Vorrichtungen 1.1;1.2 der Fig. 1 eingesetzt werden kann. Die Stützplatte 5 bildet für zumindest einige der Ausschnitte 8 zwischen dem Ausschnitt 8 und den dazu nächstliegenden der Rohröffnungen 7.1 - 7.3 einen jeweiligen Steg 9 aus. Die Stege 9 haben jeweils eine minimale Breite b, welche bei allen der Stege 9 gleich groß ist. Die Ausschnitte 8 liegen jeweils zentral zwischen drei paarweise zueinander benachbarten der Rohröffnungen 7.1 - 7.3. Die Stützplatte 5 weist Zwischenbereiche 11 auf, welche jeweils zwischen drei paarweise zueinander benachbarten der Rohröffnungen 7.1 - 7.3 ausgebildet sind. Die Ausschnitte 8 der Stützplatte 5 sind auf einen jeweiligen der Zwischenbereiche 11 beschränkt. Die Zwischenbereiche 11 sind sternförmig.

Fig. 6 zeigt eine Detailansicht einer vierten Ausgestaltung einer Stützplatte 5, wie sie in den Vorrichtungen 1.1;1.2 der Fig. 1 eingesetzt werden kann. Die Stützplatte 5 bildet für zumindest einige der Ausschnitte 8 zwischen dem Ausschnitt 8 und den dazu nächstliegenden der Rohröffnungen 7.1 - 7.4 einen jeweiligen Steg 9 aus. Die Stege 9 haben jeweils eine minimale Breite b, welche bei allen der Stege 9 gleich groß ist.

Die Stützplatte 5 weist Zwischenbereiche 11.1; 11.2 auf, welche jeweils zwischen drei paarweise zueinander benachbarten der Rohröffnungen 7.1 - 7.4 ausgebildet sind, und wobei sich zumindest einige der Ausschnitte 8 der Stützplatte 5 jeweils über mindestens zwei benachbarte der Zwischenbereiche 11.1;11.2 erstrecken. Die Zwischenbereiche 11.1,11.2 sind sternförmig.

### Bezugszeichenliste

- 1.1;1.2: Vorrichtung
- 2: Reaktorbehälter
- 3: Rohrbündel
- 4: Rohr
- 5: Stützplatte
- 6: Längsachse
- 7; 7.1 - 7.4: Rohröffnung
- 8: Ausschnitt
- 9: Steg
- 10: Durchgangsbohrung
- 11; 11.1; 11.2: Zwischenbereich
- 12: erstes Ende
- 13: zweites Ende
- 14: Verteiler
- 15: Sammler
- 16: gesättigter Dampf
- 17: Wasser leicht unter bzw. am Siedepunkt
- 18: Mantelraum
- 19: Synthesegaseinlass
- 20: vorgeheiztes Synthesegas
- 21: Gaseinlass
- 22: Produktgasauslass
- 23: Anordnung
- 24: Katalysator
- 25: Kondensator
- b: minimale Breite

## Patentansprüche

1. Vorrichtung (1.1;1.2) umfassend einen Reaktorbehälter (2), ein Rohrbündel (3) mit mehreren Rohren (4), und zumindest eine Stützplatte (5),
wobei das Rohrbündel (3) im Reaktorbehälter (2) angeordnet ist,
wobei die Stützplatte (5) quer zu einer Längsachse (6) des Reaktorbehälters (2) im Reaktorbehälter (2) angeordnet ist,
wobei jedes Rohr (4) des Rohrbündels (3) durch eine jeweilige Rohröffnung (7) der Stützplatte (5) geführt ist,
wobei die Stützplatte (5) die Rohre (4) des Rohrbündels (3) in den Rohröffnungen (7) quer zur Längsrichtung der Rohre (4) stützt,
wobei die Stützplatte (5) zwischen den Rohröffnungen (7) Ausschnitte (8) zum Fluidaustausch aufweist.

2. Vorrichtung (1.1; 1.2) nach Anspruch 1, wobei die Stützplatte (5) für zumindest einige der Ausschnitte (8) zwischen dem Ausschnitt (8) und den dazu nächstliegenden der Rohröffnungen (7) einen jeweiligen Steg (9) ausbildet, und wobei die Stege (9) jeweils eine minimale Breite (b) haben, welche bei zumindest einigen der Stege (9) gleich groß ist.

3. Vorrichtung (1.1;1.2) nach einem der vorherigen Ansprüche, wobei zumindest einige der Ausschnitte (8) jeweils zentral zwischen drei paarweise zueinander benachbarten der Rohröffnungen (7.1 - 7.3) liegen.

4. Vorrichtung (1.1; 1.2) nach einem der vorherigen Ansprüche, wobei die Stützplatte (5) Zwischenbereiche (11) aufweist, welche jeweils zwischen drei paarweise zueinander benachbarten der Rohröffnungen (7.1 - 7.3) ausgebildet sind, und wobei zumindest einige der Ausschnitte (8) der Stützplatte (5) auf einen jeweiligen der Zwischenbereiche (11) beschränkt sind.

5. Vorrichtung (1.1;1.2) nach einem der vorherigen Ansprüche, wobei zumindest einige der Zwischenbereiche (11) sternförmig sind.

6. Vorrichtung (1) nach einem der vorherigen Ansprüche, wobei die Stützplatte (5) Zwischenbereiche (11.1; 11.2) aufweist, welche jeweils zwischen drei paarweise zueinander benachbarten der Rohröffnungen (7.1 - 7.4) ausgebildet sind, und wobei sich zumindest einige der Ausschnitte (8) der Stützplatte (5) jeweils über mindestens zwei benachbarte der Zwischenbereiche (11.1;11.2) erstrecken.

7. Vorrichtung (1.1;1.2) nach einem der vorherigen Ansprüche, wobei die Stützplatte (5) alle Rohre (4) des Rohrbündels (3) umgreift.

8. Anordnung (23) zur Methanolsynthese umfassend eine Vorrichtung (1.1; 1.2) nach einem der Ansprüche 1 bis 7.

9. Verwendung einer Vorrichtung (1.1;1.2) nach einem der Ansprüche 1 bis 7 zur Methanolsynthese.

10. Verfahren zur Methanolsynthese mit einer Vorrichtung (1.1;1.2) nach einem der Ansprüche 1 bis 7, wobei Reaktionsedukte für die Methanolsynthese durch die Rohre (4) des Rohrbündels (3) geleitet werden und ein Kühlmedium außerhalb der Rohre (4) des Rohrbündels (3) durch den Reaktorbehälter (2) geleitet wird,
oder
wobei Reaktionsedukte für die Methanolsynthese außerhalb der Rohre (4) des Rohrbündels (3) durch den Reaktorbehälter (2) geleitet werden und ein Kühlmedium durch die Rohre (4) des Rohrbündels (3) geleitet wird.
